# EUROPEAN PATENT APPLICATION

(11) **EP 4 472 186 A1**
(43) Date of publication of application: **04.12.2024**
(21) Application number: 23747392.1
(22) Date of filing: 27.01.2023
(51) Int. Cl.: H04M 1/72436, A63B 24/00, A63B 71/06

(54) **ELECTRONIC DEVICE FOR PROVIDING CHAT MESSAGES AND EXERCISE STATE INFORMATION TOGETHER AND CONTROL METHOD THEREFOR**

(30) Priority: 28.01.2022 KR 20220013160; 11.02.2022 KR 20220018374
(71) Applicant: Samsung Electronics Co., Ltd., Suwon-si, Gyeonggi-do 16677 (KR)
(72) Inventor: PARK, Sangil, Suwon-si Gyeonggi-do 16677 (KR); KIM, Kyeol, Suwon-si Gyeonggi-do 16677 (KR); KIM, Dongmyung, Suwon-si Gyeonggi-do 16677 (KR); KIM, Ayoung, Suwon-si Gyeonggi-do 16677 (KR)
(74) Representative: Walaski, Jan Filip
(86) International application number: PCT/KR2023/001289
(87) International publication number: WO 2023/146351

(57) **Abstract**

Disclosed are an electronic device for providing chat messages and exercise state information together and a control method therefor. The electronic device according to an embodiment of the present document comprises a touch screen display and at least one processor. The at least one processor may: display a first execution screen of a first application on the touchscreen display on the basis of a chat session set between the electronic device and at least one external electronic device, wherein the first execution screen includes a first visual element indicating a user of the at least one external electronic device and an exercise progress situation of a user of the electronic device, and a second visual element for outputting a guidance message for guiding exercise progress; receive a selection input for the second visual element; and display a second execution screen of the first application on the touchscreen display on the basis of the selection input, wherein the second screen includes at least one of the first visual element or the guidance message.

## Description

### [Technical Field]

The present document relates to an electronic device that provides both chat messages and exercise state information and a method of controlling the same.

### [Background Art]

Various services and additional functions provided through electronic devices, for example, portable electronic devices such as smart phones, are gradually increasing. To enhance the utility value of these electronic devices and satisfy the needs of various users, communication service providers or electronic device manufacturers are competitively developing electronic devices to provide various functions and differentiate themselves from other companies. Accordingly, the various functions provided through the electronic devices are becoming increasingly sophisticated.

### [Detailed Description of the Invention]

### [Technical Problem]

In conventional applications (e.g., messenger applications), only general chat functions (e.g., sending and receiving chat messages) are provided. Thus, unless multiple users share their current situations via chat messages in a group chat room, it is difficult to know what others are currently doing. For example, according to the functions provided by conventional applications (e.g., messenger applications), when the user of an electronic device is performing exercise with other users, it is difficult for the user to know the current exercise status of the other users or how the user's exercise status compares to that of the other users.

According to various embodiments of the present disclosure, an electronic device can be provided that allows the user to feel as if they are exercising with other users by offering information on the exercise progress situation of the other users along with chat messages, and that enables the user of the electronic device to be motivated to continue exercising.

According to various embodiments of the present disclosure, a control method of an electronic device can be provided that allows the user to feel as if they are exercising with other users by offering information on the exercise progress situation of the other users along with chat messages, and that enables the user of the electronic device to be motivated to continue exercising.

### [Technical Solution]

In accordance with an aspect of the present disclosure, an electronic device includes a touchscreen display and at least one processor, wherein the at least one processor is configured to: display a first execution screen of a first application on the touchscreen display based on a chat session set between at least one external electronic device and the electronic device, the first execution screen including a first visual element indicating a user of the at least one external electronic device and an exercise progress situation of a user of the electronic device, and a second visual element for outputting a guidance message for guiding exercise progress; receive a selection input for the second visual element; and display a second execution screen of the first application on the touchscreen display based on the selection input, the second screen including at least one of the first visual element and the guidance message.

In accordance with another aspect of the present disclosure, a method of controlling an electronic device includes displaying a first execution screen of a first application on a touchscreen display of the electronic device based on a chat session set between at least one external electronic device and the electronic device, wherein the first execution screen includes a first visual element indicating a user of the at least one external electronic device and an exercise progress situation of a user of the electronic device, and a second visual element for outputting a guidance message for guiding exercise progress; receiving a selection input for the second visual element; and displaying a second execution screen of the first application on the touchscreen display based on the selection input, the second screen including at least one of the first visual element and the guidance message.

### [Advantageous Effects]

According to various embodiments of the present disclosure, an electronic device can be provided that allows the user to feel as if they are exercising with other users by offering information on the exercise progress situation of the other users along with chat messages, and that enables the user of the electronic device to be motivated to continue exercising.

Effects according to various embodiments are not limited to the effects described above, and it is clear to those skilled in the art that various effects are inherent in the present disclosure.

### [Brief Description of Drawings]

FIG. 1 is a block diagram illustrating an electronic device in a network environment according to various embodiments of the present disclosure.
FIG. 2 is an exemplary diagram illustrating a function or operation of an electronic device providing a chat room including information on an exercise progress situation according to an embodiment of the present disclosure.
FIGS. 3A, 3B, 3C, 3D, and 3E are diagrams illustrating the function or operation described in FIG. 2 through a graphical user interface.
FIG. 4 is an exemplary diagram illustrating a function or operation of an electronic device generating (e.g., setting) a chat room according to an embodiment of the present disclosure.
FIGS. 5A, 5B, 5C, 5D, and 5E are diagrams illustrating the function or operation described in FIG. 4 through a graphical user interface.
FIGS. 6A, 6B, and 6C are diagrams illustrating an example of a chat room in which an exercise type according to an embodiment of the present disclosure is set to "walking".
FIGS. 7A, 7B, and 7C are diagrams illustrating an example of a chat room in which an exercise type is set to "tabata" according to an embodiment of the present disclosure.
FIGS. 8A and 8B ae diagrams illustrating an example of a chat room in which an exercise type is set to "diet" according to an embodiment of the present disclosure.

### [Mode for Carrying out the Invention]

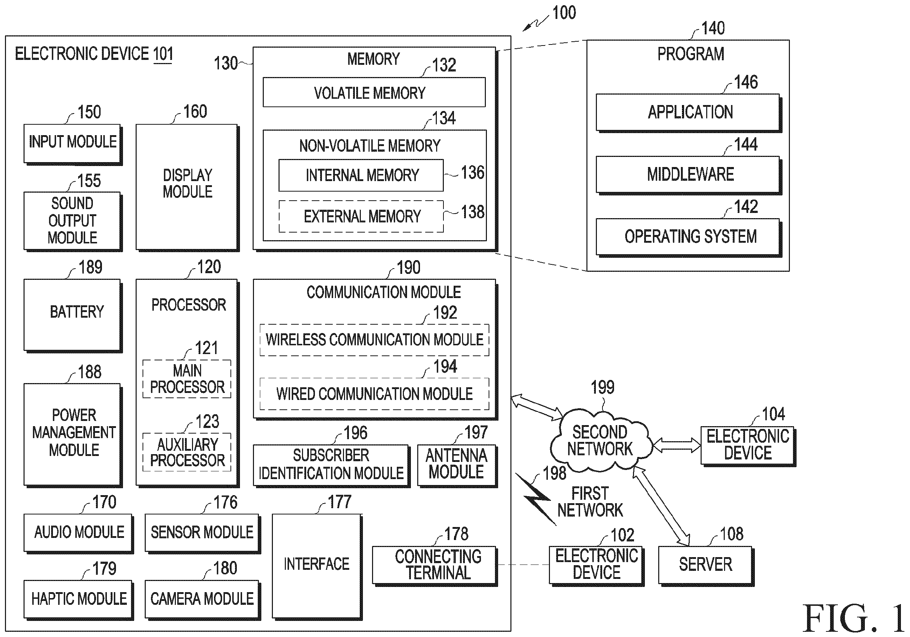

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

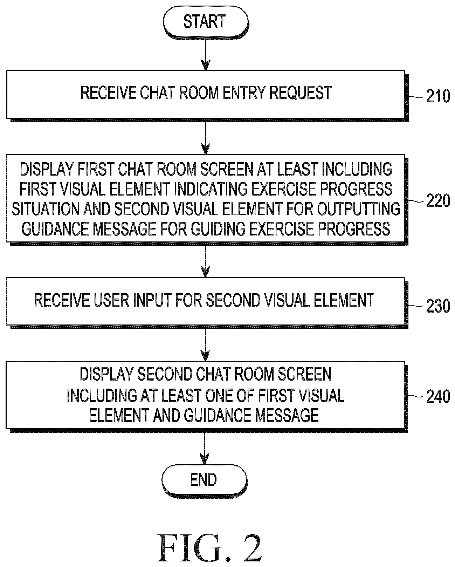

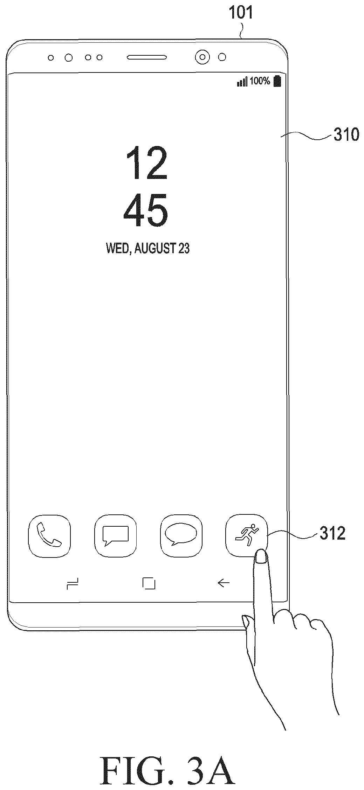

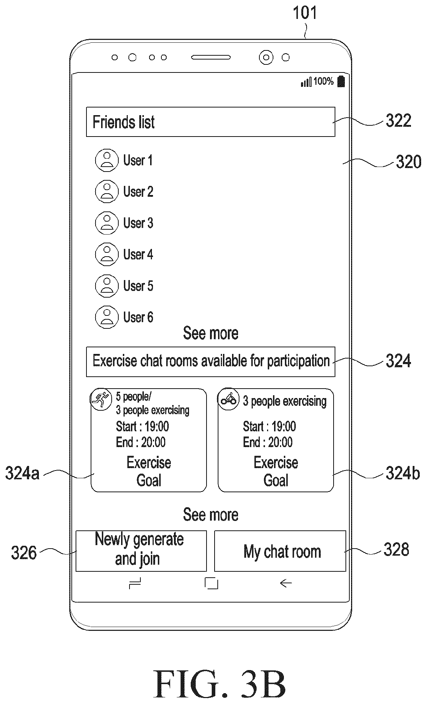

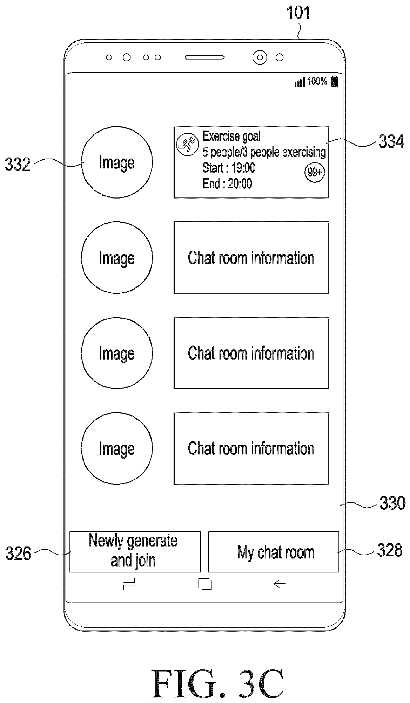

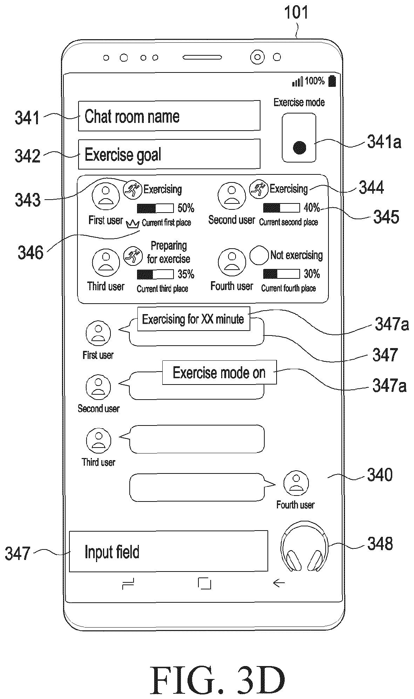

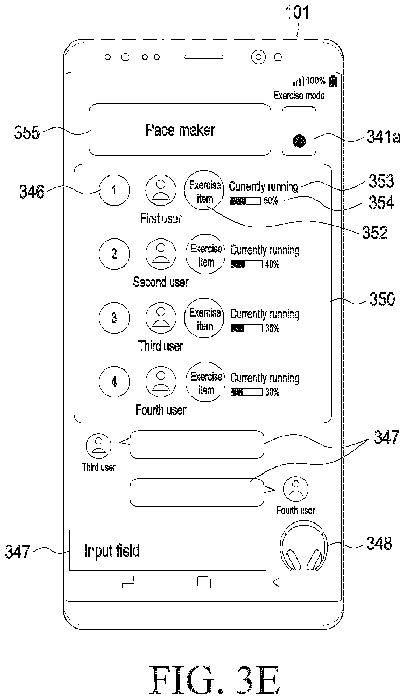

FIG. 2 is an exemplary diagram illustrating a function or operation of an electronic device providing a chat room including information on an exercise progress situation according to an embodiment of the present disclosure. FIGS. 3A, 3B, 3C, 3D, and 3E are diagrams illustrating the function or operation described in FIG. 2 through a graphical user interface.

Referring to FIG. 2, an electronic device 101 (e.g., a processor 120) may receive a chat room entry request in operation 210. The electronic device 101 according to the present disclosure may receive information on a chat room (e.g., a first chat room 324a of FIG. 3B and/or a second chat room 324b of FIG. 3B) available for participation or a selection input on a chat room (e.g., a third chat room 334 of FIG. 3C) in which the user is currently participating, included in a first execution screen 320, thereby receiving the chat room entry request. In the present document, the term "chat room" may also be referred to as a "chat session" or "group session". The "chat room" mentioned in the present document is an example of a "group session" that includes multiple users, and is not necessarily limited to a specific session for the purpose of providing chat messages. For example, in the case of a group session that includes users of a plurality of electronic devices, various embodiments mentioned in the present document can be applied even if chat messages are not provided through the group session. In addition, various embodiments mentioned in the present document may be provided as a specific application itself, or may be provided as one function among a plurality of functions included in the specific application. In order to receive the chat room entry request (e.g., before receiving the chat room entry request), the electronic device 101 according to an embodiment of the present document may receive an execution request (e.g., a touch input to a specific application icon 312 displayed on a home screen 310) of a specific application (e.g., a messenger application or Samsung^{®} Health^{™} application), as shown in FIG. 3A. The electronic device 101 according to an embodiment of the present document may display a first execution screen 320 of a specific application (e.g., a messenger application or Samsung^{®} Health^{™} application) as shown in FIG. 3B on a touchscreen display (e.g., the display module 160), based on an execution request shown in FIG. 3A. The first execution screen 320 according to an embodiment of the present document may include a first list 322 of a user of at least one external electronic device, based on contact information stored in the electronic device 101. The electronic device 101 according to an embodiment of the present document may receive a selection input for at least one other user included in the first list 322, thereby inviting users belonging to a newly generated chat room (e.g., a third execution screen 340 of FIG. 3D). Alternatively, the electronic device 101 according to an embodiment of the present document may add at least one other user to be included in the first list 322 through various methods, such as scanning a quick response (QR) code or searching for ID (e.g., the phone number of the other party's device), and invite users belonging to the newly generated chat room (e.g., the third execution screen 340 of FIG. 3D). The first execution screen 320 according to an embodiment of the present document may include a second list 324 for a chat room (e.g., a first chat room 324a and/or a second chat room 324b) in which the user of the electronic device 101 can participate. According to an embodiment of the present document, information on the chat room (e.g., the first chat room 324a and/or the second chat room 324b) in which the user can participate may be recommended by the electronic device 101 or an external electronic device (e.g., a server) based on the type of exercise the user of the electronic device 101 is currently performing or the history of the type of exercise the user has performed in the past. Alternatively, the electronic device 101 or the external electronic device (e.g., the server) according to an embodiment of the present document may recommend (e.g., provide information on the first chat room 324a when a specific time is reached) information on the chat room (e.g., the first chat room 324a and/or the second chat room 324b) in which the user can participate based on the results trained by an artificial intelligence model. The artificial intelligence model according to an embodiment of the present document may include a plurality of artificial neural network layers. The artificial neural network according to an embodiment of the present document may be a deep neural network (DNN), a convolutional neural network (CNN), a recurrent neural network (RNN), a restricted Boltzmann machine (RBM), a deep belief network (DBN), a bidirectional recurrent deep neural network (BRDNN), a deep Q-network, or a combination of two or more of the above-mentioned networks, but is not limited to the above examples. The first execution screen 320 according to an embodiment of the present document may further display information (e.g., a running icon and/or a cycling icon) on the exercise type set for the chat room (e.g., the first chat room 324a and/or the second chat room 324b) available for participation, together with information on the chat room available for participation (e.g., the first chat room 324a and/or the second chat room 324b). The exercise type (e.g., running and/or cycling) according to an embodiment of the present document may be set by the external electronic device (e.g., a server to which the electronic device 101 is connected) based on an input of a user who generates a target chat room (e.g., the first chat room 324a), or may be set by the external electronic device (e.g., the server to which the electronic device 101 is connected) based on biometric information acquired by the electronic device (e.g., a wearable device) of the user who generated the target chat room (e.g., the first chat room 324a). As to the information (e.g., running icon and/or cycling icon) on the exercise type according to an embodiment of the present document, for example, when a plurality of users included in a chat room are performing different exercises, information on the type of exercise being performed by the user who generated the target chat room (e.g., the first chat room 324a) may be displayed as a representative. According to an embodiment of the present document, the information on the chat room (e.g., the first chat room 324a and/or the second chat room 324b) available for participation may include at least one of the start time of the exercise, the end time of the exercise, the goal of the exercise (e.g., losing 10 kg or walking 10 km), or the number of participants in the chat room. The start time of the exercise, the end time of the exercise, the goal of the exercise (e.g., losing 10 kg or walking 10 km) according to an embodiment of the present disclosure may be set by the user who generated the target chat room (e.g., the first chat room 324a). However, according to another embodiment of the present document, the start time of the exercise, the end time of the exercise (estimated end time of exercise), the goal of the exercise (e.g., losing 10 kg or walking 10 km) according to an embodiment of the present disclosure may be set (e.g., changed) by any user belonging to the chat room. According to an embodiment of the present document, the start time of the exercise and the end time of the exercise may be displayed when the user of the electronic device 101 and/or at least one user included in the chat room is exercising. According to an embodiment of the present document, the estimated end time of exercise may be estimated based on exercise history information of a user who started exercising (e.g., a user who first started exercising in the chat room). Alternatively, various estimation methods using artificial intelligence models can be applied. The first execution screen 320 according to an embodiment of the present document may include a first menu 326 for generating a chat room and/or a second menu 328 for displaying a list of a chat room in which the user of the electronic device 101 is currently participating. The first execution screen 320 according to an embodiment of the present document may include a screen when the first menu 326 is selected by the user. Referring to FIG. 3C, the electronic device 101 according to an embodiment of the present document may display the second execution screen 330 on the touchscreen (e.g., the display module 160) based on the second menu 328 being selected by the user. The second execution screen 330 according to an embodiment of the present document may include an image 332 set by the user who generated the target chat room (e.g., the third chat room 334). The second execution screen 330 according to an embodiment of the present document may include at least one of the type of the exercise set by the user who generated the type of the exercise (e.g., the target chat room {e.g., the third chat room 334}) set for the chat room, information (e.g., running icon) on the type of the exercise identified by the electronic device of the user who generated the target chat room (e.g., the third chat room 334) based on biometric information of the user who generated the target chat room (e.g., the third chat room 334) or the external electronic device (e.g., the server), the goal of the exercise (e.g., losing 10 kg or walking 10 km), the start time of the exercise, the end time of the exercise, or the number of new chat messages. When a selection input for the target chat room (e.g., the third chat room 334) is received from the user, the electronic device according to an embodiment of the present document may display a chat room (e.g., the third execution screen 340) for the selected target chat room (e.g., the third chat room 334).

In operation 220, the electronic device 101 according to an embodiment of the present document may display a first chat room screen (e.g., the third execution screen 340) including at least one of a first visual element (e.g., a third object 345) indicating an exercise progress situation and a second visual element (e.g., a fifth object 341a) for outputting a guidance message for guiding exercise progress. Referring to FIG. 3D, the third execution screen 340 according to an embodiment of the present document may include at least one of a chat room name 341, an exercise goal 342, information indicating the type of exercise in which each of at least one user included in the chat room is performing (e.g., a first object 343), information indicating the exercise situation of each user (e.g., "currently exercising," "preparing to exercise," or "not exercising") (e.g., a second object 344), information on the exercise progress situation to date (e.g. a third object 345), and information on the relative ranking of the at least one user included in the chat room, identified based on the exercise progress situation to date (e.g., a fourth object 346). The chat room name 341 according to an embodiment of the present disclosure may be a name set by the user who generated the target chat room (e.g., the chat room corresponding to the third execution screen 340). The exercise goal 342 according to an embodiment of the present document may be a goal set by the user who generated the target chat room (e.g., the chat room corresponding to the third execution screen 340). The information indicating the type of exercise in which each of at least one user included in the chat room is performing (e.g., the first object 343) may be pre-designated (e.g., the first object 343 is automatically generated and displayed when the user enters the target chat room) depending on the chat room, or may be determined and displayed according to the type of user's exercise identified based on the user's biometric information (e.g., after the user enters the target chat room, the first object 343 is not displayed for a certain period of time, then the user's biometric information is acquired for a certain period of time and the first object 343 is and generated and displayed according to the type of exercise determined based on the acquired biometric information). According to an embodiment of the present disclosure, the exercise situation of each user (e.g., "currently exercising," "preparing to exercise," or "not exercising") may be identified by the electronic device 101 or the external electronic device (e.g., a server) using the user's biometric information acquired by the electronic device 101 of the user and/or the second electronic device (e.g., a wearable device). According to an embodiment of the present disclosure, when the exercise situation is identified by the external electronic device (e.g., a server), data on the identified exercise situation may be provided to each electronic device included in the chat room. Alternatively, the information indicating the exercise situation of each user (e.g., "currently exercising," "preparing to exercise," or "not exercising") according to an embodiment of the present disclosure may be displayed in the chat room through a user's selection for a visual object (e.g., a switch) displayed on the third execution screen 340. For example, on the third execution screen 340 according to an embodiment of the present document, an activation switch for each of "currently exercising," "preparing to exercise," and/or "not exercising" may be displayed. According to an embodiment of the present disclosure, the electronic device 101 may display information (e.g., the second object 344) indicating the exercise situation based on a user's selection input to each switch. According to an embodiment of the present disclosure, the information on the exercise progress situation to date (e.g. the third object 345) (e.g., 50%) may be provided based on the accumulated results of exercising from the time the chat room was generated to the present. The information on the exercise progress situation according to an embodiment of the present disclosure may be identified by the electronic device 101 to be provided to the external electronic device (e.g., the server), or may be identified by the external electronic device (e.g., the server). According to an embodiment of the present disclosure, the information (e.g., the fourth object 346) on the relative ranking of the at least one user included in the chat room may be identified and provided by the electronic device 101 or the external electronic device (e.g., server) based on the exercise progress situation to the present. The third execution screen 340 according to an embodiment of the present disclosure may further include a visual element (e.g., a fifth object 341a) for activating a chat message 347 and a function (e.g., "exercise mode") for receiving time 347a elapsed from the start of exercise and a guidance message (e.g., a guidance message 355); a visual element (e.g., a sixth object 347b) indicating whether the function (e.g., "exercise mode") for receiving the guidance message (e.g., the guidance message 355) is activated; and a visual element (e.g., a seventh object 349) for switching to an input window 348 for inputting text messages or a voice mode (e.g., an operation mode for inputting text as a voice and/or an operation mode for outputting a text message sent by the other party as a voice). According to an embodiment of the present disclosure, the visual element (e.g., the sixth element 347b) indicating whether the function (e.g., "exercise mode") for receiving the time 347a elapsed from the start of exercise and the guidance message (e.g., the guidance message 355) is activated may be displayed around the text message 347 as shown in FIG. 3B. However, because of this, the display position of the visual element (e.g., the sixth object 347b) indicating whether the function (e.g., "exercise mode") for receiving the time 347a elapsed from the start of exercise and the guidance message (e.g., the guidance message 355) is activated is not limited. For example, the visual element (e.g., the sixth object 347b) indicating whether the function (e.g., "exercise mode") for receiving the time 347a elapsed from the start of exercise and the guidance message (e.g., the guidance message 355) is activated may be separated from the text message 347 and displayed. When the "voice mode" is activated according to an embodiment of the present document, voice conversation may be possible between electronic devices for which "voice mode" is activated. In addition, when "voice mode" according to an embodiment of the present document is activated, the guidance message (e.g., the guidance message 355) may be output as a voice. The third execution screen 340 according to an embodiment of the present document may include a representative image set by each user. When a user's selection input for the representative image is detected, the electronic device 101 according to an embodiment of the present document may display a user's exercise history corresponding to the representative image, at least one exercise type performed by the user, the number of exercises, and the like.

In operation 230, the electronic device101 according to an embodiment of the present disclosure may receive a user input for a second visual element (e.g., the fifth object 341a). The user input according to an embodiment of the present document may include, for example, a touch input to the second visual element (e.g., the fifth object 341a), but this does not limit the type of user input.

In operation 240, the electronic device 101 according to an embodiment of the present document may display a second chat room screen (e.g., a fourth execution screen 350) including at least one of the first visual element (e.g., the third object 345) and a guidance message on the touchscreen display (e.g., the display module 160) based on the user input in operation 230. Referring to FIG. 3E, the second chat room screen (e.g., the fourth execution screen 350) according to an embodiment of the present document may include a guidance message (e.g., the guidance message 355). The guidance message (e.g., the guidance message 355) according to an embodiment of the present disclosure may include a message related to adjustment of each user's exercise pace. For example, the guidance message (e.g., the guidance message 355) according to an embodiment of the present disclosure may include a message indicating a current status (e.g., at least one of elapsed exercise time, achievement rate, current ranking, and calorie consumption) at each designated time after starting the exercise. The guidance message (e.g., the guidance message 355) according to an embodiment of the present disclosure may include a message such as "n % remains until the current exercise goal achievement rate," through comparison with a target value set through the current exercise situation and the exercise goal. The guidance message (e.g., the guidance message 355) according to an embodiment of the present disclosure may identify a difference in the exercise goal achievement rates between the highest and lowest rankings, and may include a message such as "The difference with first place is currently increasing by n %" when the difference in the exercise goal achievement rates between the highest and lowest rankings increases by a threshold value. The guidance message (e.g., the guidance message 355) according to an embodiment of the present disclosure may include a message such as "currently changed from n ranking to m ranking" when there is a change in the rankings related to exercise progress. According to an embodiment of the present disclosure, a function or operation of comparing the current exercise situation and the target value set through the exercise goal may be performed by the electronic device 101 or the external electronic device (e.g., the server). In the same manner, the function or operation of identifying the difference in the exercise goal achievement rates between the highest and lowest rankings and the function or operation of identifying whether there is the change in rankings related to exercise progress may be performed by the electronic device 101 or the external electronic device (e.g., the server). The fourth execution screen 350 according to an embodiment of the present document may include at least one of the fifth object 341a, information 345 on the current ranking, information 352 on exercise items, information 353 on exercise type and/or current exercise status (e.g., "running, currently exercising" or "running, preparing for exercise"), information 354 on exercise progress made since the chat room was generated (or when the electronic device was first included in the chat room) to date (e.g., 50 %), the chat message 347, and the visual element 349 for switching to the input window 348 or the exercise mode.

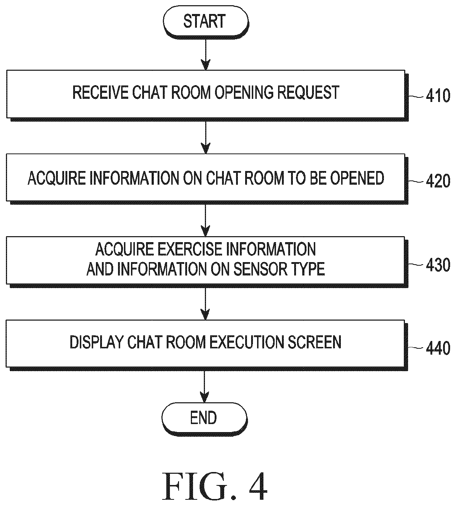

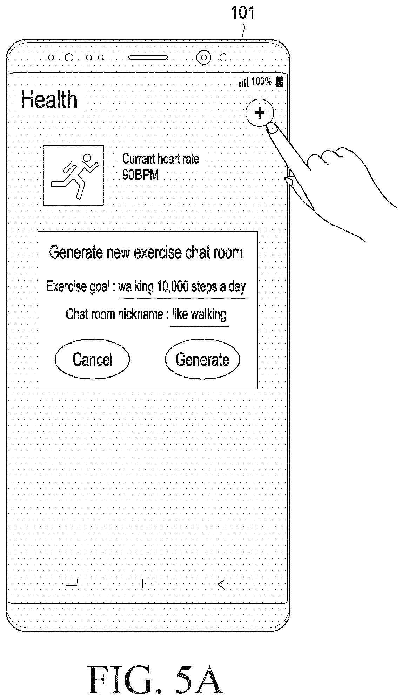

FIG. 4 is an exemplary diagram illustrating a function of operation of an electronic device generating (e.g., setting) a chat room according to an embodiment of the present disclosure. FIGS. 5A to 5E are diagrams illustrating the function or operation described in FIG. 4 through a graphical user interface.

Referring to FIG. 4, the electronic device 101 according to an embodiment of the present disclosure may receive a chat room opening (e.g., generating) request in operation 410. As shown in FIG. 5A , the electronic device 101 according to an embodiment of the present document may receive the chat room opening request by receiving a user's selection input for a specific object. As shown in FIG. 5A, when receiving the user's selection input for the specific object, the electronic device 101 according to an embodiment of the present document may display a screen (e.g., a pop-up window) for receiving the exercise goal of a chat room to be opened and the nickname of the chat room. According to an embodiment of the preset disclosure, when the screen (e.g., the pop-up window) for receiving the exercise goal of the chat room to be opened and the nickname of the chat room, a recommended goal and recommended nickname may be displayed together as shown in FIG. 5A. However, according to another embodiment of the present document, the recommended goal and recommended nickname may not be displayed.

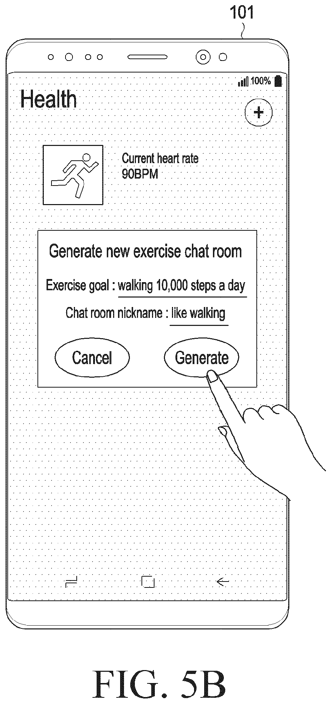

In operation 420, the electronic device 101 according to an embodiment of the present disclosure may acquire information on the chat room to be opened. As shown in FIG. 5B, the electronic device according to an embodiment of the present disclosure may use the recommended goal and recommended nickname as is to generate the chat room, or may input a different exercise goal and nickname that are different from the recommended goal and recommended nickname. The electronic device 101 according to an embodiment of the present disclosure may receive a user's confirmation input (e.g., a touch input on a "generate" menu) for the chat room opening request, as shown in FIG. 5B.

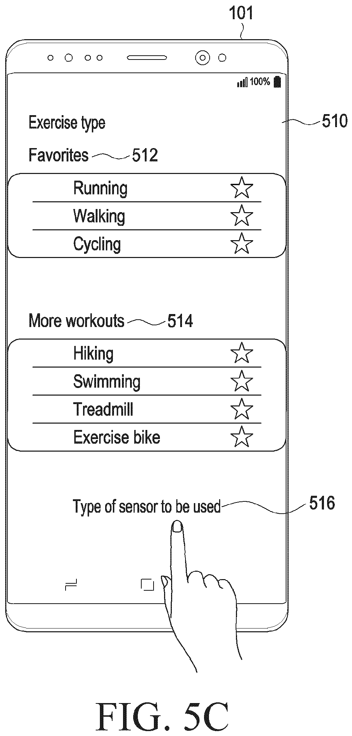

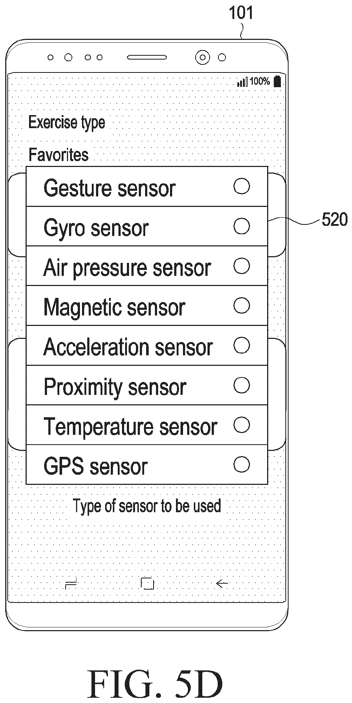

In operation 430, the electronic device 101 according to an embodiment of the present disclosure may acquire exercise information and information on a sensor type. The electronic device 101 according to an embodiment of the present disclosure may display a list 512 of at least one exercise type preferred by the user of the electronic device 101 (e.g., has a history in which the corresponding exercise has been performed in the past) and a list 514 related to at least one other exercise type, as shown in FIG. 5C. The electronic device 101 according to an embodiment of the present disclosure may receive a user's input for selecting one exercise type from among the lists 512 and 514 related to the exercise types. The electronic device 101 according to an embodiment of the present document may set the selected exercise type as an exercise type representing the chat room. For example, the electronic device 101 according to an embodiment of the present document may display an icon (e.g., a running icon in FIG. 3B) representing the selected exercise type (e.g., running) as one information on the chat room. The electronic device 101 according to an embodiment of the present document may receive a user's input for setting the type of a sensor to be used in order to determine whether the user of the electronic device 101 is performing exercise (e.g., determine whether the user starts to exercise) and/or identify the exercise progress situation of the user (e.g., identify how many steps the user has walked). When receiving the user's input for setting the type of the sensor, the electronic device 101 according to an embodiment of the present document may display at least one sensor list 520, as shown in FIG. 5D. The at least one sensor list according to an embodiment of the present document may include, but is not limited to, a gesture sensor, a gyro sensor, an air pressure sensor, a magnetic sensor, an acceleration sensor, a temperature sensor, and/or a GPS sensor. The at least one sensor included in the sensor list 520 according to an embodiment of the present disclosure may include a sensor provided in the electronic device 101 and/or an external electronic device (e.g., a wearable device) operably connected to the electronic device 101. When the at least one sensor is selected, the electronic device 101 according to an embodiment of the present disclosure may use the sensor provided in the electronic device 101 and/or the external electronic device (e.g., a wearable device) operably connected to the electronic device 101 to determine whether the user of the electronic device 101 is performing exercise or identify the exercise progress situation. For example, when the "acceleration sensor" is selected by the user, data sensed from the acceleration sensor of the wearable device (e.g., a smart watch) operably connected to the electronic device 101 may be received to determine whether the user of the electronic device 101 is performing exercise or identify the exercise progress situation.

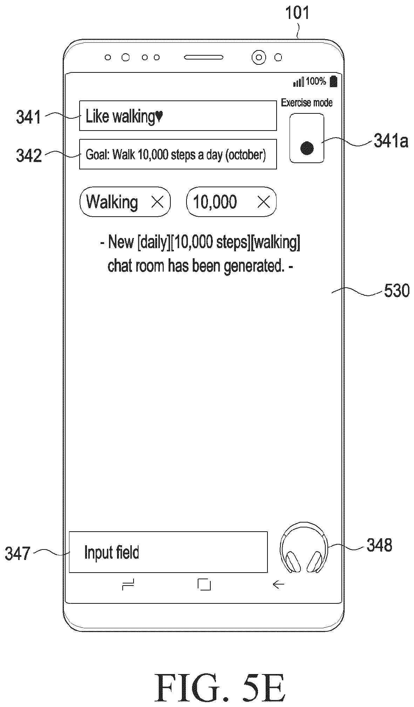

In operation 440, the electronic device 101 according to an embodiment of the present disclosure may display a chat room execution screen. The electronic device 101 according to an embodiment of the present document may display a chat room execution screen 530 as shown in FIG. 5E. The chat room execution screen 530 according to an embodiment of the present document may include at least one of a chat room name 341, a second visual element (e.g., the fifth object 341a) for outputting a guidance message for guiding exercise progress, an exercise goal 342, and a visual element (e.g., the seventh object 349) for switching to an input window 348 for inputting text messages or a voice mode (e.g., an operation mode for inputting text as a voice and/or an operation mode for outputting a text message sent by the other party as a voice). The chat room execution screen 530 may further include recommended search terms (e.g., "walking", "10,000") or words searched by the user.

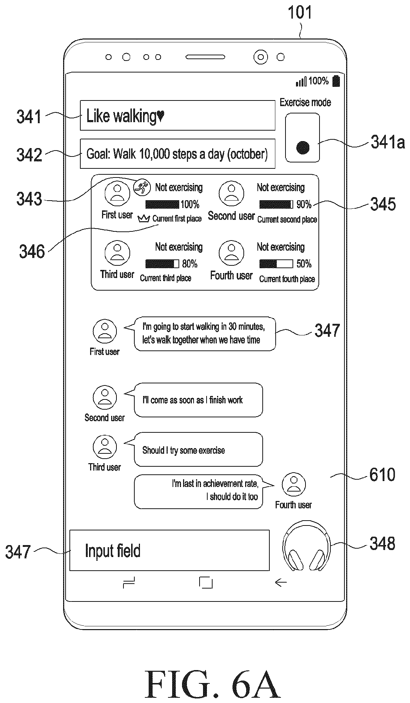

FIGS. 6A to 6C are diagrams illustrating an example of a chat room in which an exercise type according to an embodiment of the present disclosure is set to "walking".

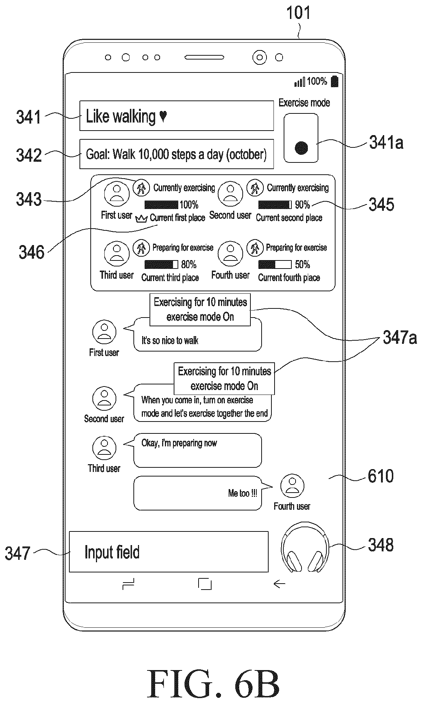

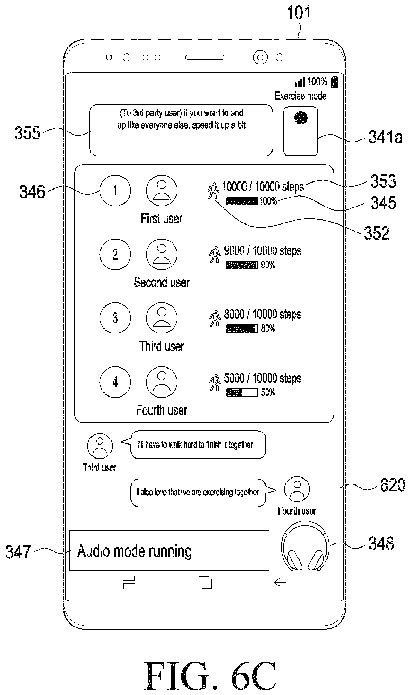

Referring to FIG. 6A, the electronic device 101 according to an embodiment of the present disclosure may enter (e.g., display a chat room screen {e.g., the fifth execution screen 610}) a chat room according to a user's input. In FIG. 6A, since the users included in the chat room are not currently performing walking exercise, information (e.g., the first object 343) indicating the type of exercise each of at least one user is performing may include a circular shape that does not contain a specific icon, as shown in FIG. 6A. Referring to FIG. 6B, the electronic device 101 according to an embodiment of the present disclosure may update and display a chat room screen (e.g., the fifth execution screen 610) according to the user's exercise performance status (e.g., "currently exercising" or "preparing for exercise"). Referring to FIG. 6C, when the electronic device 101 enters the exercise mode from the normal mode by the user of the electronic device 101, the electronic device 101 according to an embodiment of the present document may display a guidance message 355 as shown in FIG. 6C.

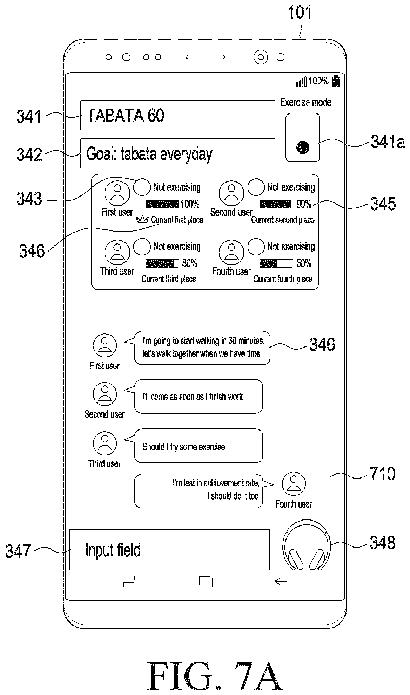

FIGS. 7A to 7C are diagrams illustrating an example of a chat room in which an exercise type is set to "tabata" according to an embodiment of the present disclosure.

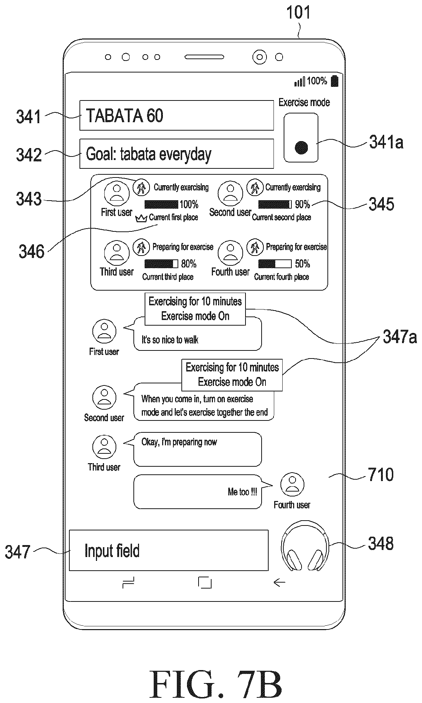

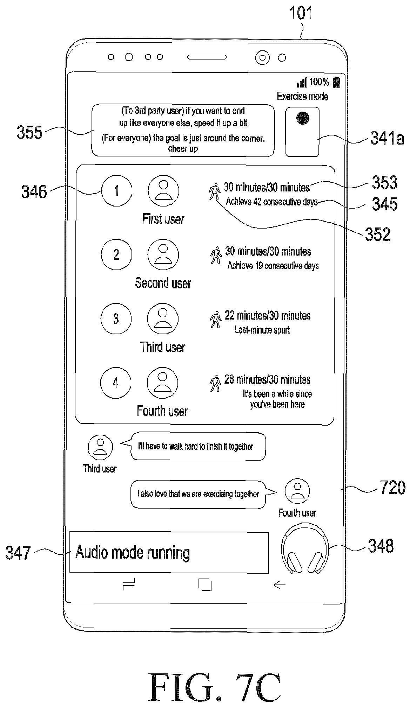

Referring to FIG. 7A, the electronic device 101 according to an embodiment of the present document may enter (e.g., display a chat room screen {e.g., the sixth execution screen 710}) a chat room according to a user's input. In FIG. 7A, since the users included in the chat room are not currently performing tabata exercise, information indicating the type of the exercise each of at least one user is performing (e.g., the first object 343) may include a circular shape that does not contain a specific icon, as shown in FIG. 7A. Referring to FIG. 7B, the electronic device 101 according to an embodiment of the present disclosure may update and display a chat room screen (e.g., the sixth execution screen 710) according to the user's exercise performance status (e.g., "currently exercising" or "preparing for exercise"). Referring to FIG. 7C, when the electronic device 101 enters the exercise mode from the normal mode by the user of the electronic device 101, the electronic device 101 according to an embodiment of the present document may display the guidance message 355 as shown in FIG. 7C. In the exercise mode, the electronic device 101 according to an embodiment of the present document may display information 352 on exercise items and information 353 on exercise type and/or current exercise state (e.g., "current performance time: 30 minutes/target value: 30 minutes"). In FIG. 7C, a case in which "audio mode" is executed is exemplarily shown.

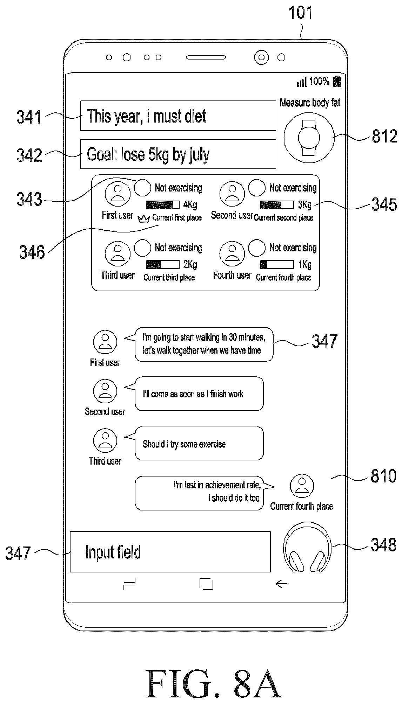

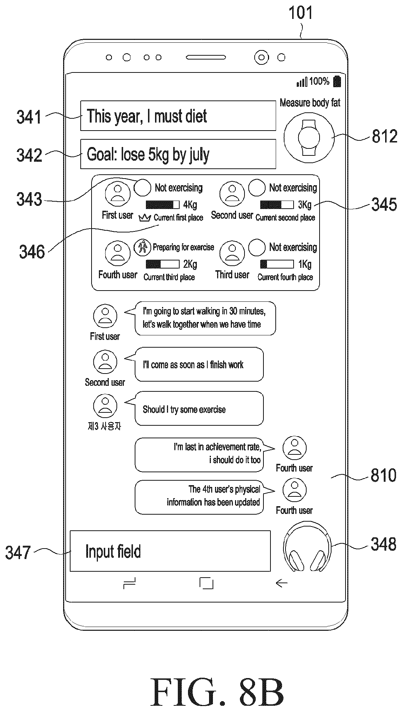

FIGS. 8A and 8B are diagrams illustrating an example of a chat room in which an exercise type is set to "diet" according to an embodiment of the present disclosure.

Referring to FIG. 8A, the electronic device 101 according to an embodiment of the present disclosure may enter (e.g., display a chat room screen {e.g., the seventh execution screen 810}) a chat room according to a user's input. In FIG. 8A, since the users included in the chat room are not currently performing a specific exercise, information (e.g., the first object 343) indicating the type of exercise each of at least one user is performing may include a circular shape that does not contain a specific icon, as shown in FIG. 8A. The electronic device 101 according to an embodiment of the present disclosure may display an object 812 for measuring biometric information (e.g., body fat) by an external electronic device (e.g., a wearable device) operably connected to the electronic device 101. When receiving, from the user, a selection input for the object 812 for measuring the biometric information (e.g., body fat) by the external electronic device (e.g., the wearable device) operably connected to the electronic device 101, the electronic device 101 according to an embodiment of the present disclosure may request sensing of the biometric information from the external electronic device (e.g., the wearable device). The electronic device 101 according to an embodiment of the present disclosure may receive the sensed biometric information from the external electronic device (e.g., the wearable device), and display the received biometric information or information (e.g., the amount of body fat reduction) generated based on the received biometric information. In FIG. 8B, an embodiment in which information (e.g., the first object 343) indicating the exercise type each of the at least one user is performing is updated and displayed when the user of the electronic device 101 is "preparing for exercise" is exemplarily shown.

An electronic device (e.g., the electronic device 101 of FIG. 1) according to an embodiment of the present disclosure may include a touchscreen display (e.g., the display module 160) and at least one processor (e.g., the processor 120 of FIG. 1), wherein the at least one processor is configured to: display a first execution screen of a first application on the touchscreen display based on a chat session set between at least one external electronic device and the electronic device, the first execution screen including a first visual element indicating a user of the at least one external electronic device and an exercise progress situation of a user of the electronic device, and a second visual element for outputting a guidance message for guiding exercise progress; receive a selection input for the second visual element; and display a second execution screen of the first application on the touchscreen display based on the selection input, the second screen including at least one of the first visual element and the guidance message.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment of the disclosure, each component (e.g., a module or a program) of the above-described components may include a single entity or multiple entities, and some of the multiple entities may be separately disposed in different components. According to an embodiment of the disclosure, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to an embodiment of the disclosure, operations performed by the module, the program, or another component may be carried out sequentially, in parallel, repeatedly, or heuristically, or one or more of the operations may be executed in a different order or omitted, or one or more other operations may be added.

## Claims

1. An electronic device comprising:
a touchscreen display; and
at least one processor,
wherein the at least one processor is configured to:
display a first execution screen of a first application on the touchscreen display based on a chat session set between at least one external electronic device and the electronic device, the first execution screen comprising a first visual element indicating a user of the at least one external electronic device and an exercise progress situation of a user of the electronic device, and a second visual element for outputting a guidance message for guiding exercise progress;
receive a selection input for the second visual element; and
display a second execution screen of the first application on the touchscreen display based on the selection input, the second screen comprising at least one of the first visual element and the guidance message.

2. The electronic device of claim 1, wherein the first execution screen further comprises a third visual element indicating a current exercise status of a user of the at least one external electronic device.

3. The electronic device of claim 1, wherein the first execution screen further comprises a text message of the user of the at least one external electronic device received through the chat session and a fourth visual object indicating whether the user of the at least one external electronic device is receiving the guidance message.

4. The electronic device of claim 1, wherein the first execution screen further comprises a fifth visual object indicating ranking information according to an exercise progress situation of the user of the at least one external electronic device.

5. The electronic device of claim 1, wherein the at least one processor is further configured to identify a difference between a predetermined target value and a current exercise status of the user of the electronic device, and provide information on the identified difference as the guidance message.

6. The electronic device of claim 1, wherein the at least one processor is further configured to provide the guidance message, based on ranking information according to exercise progress situations of the user of the at least one external electronic device and the user of the electronic device.

7. The electronic device of claim 1, wherein the first execution screen comprises a chat room based on the chat session, and
wherein the at least one processor is configured to further display a type of an exercise for generating the chat room and a third execution screen for setting at least one sensor for acquiring biometric information of the user according to the type of the exercise.

8. The electronic device of claim 1, wherein the first execution screen further comprises a sixth visual element indicating an exercise performance time of the user of the at least one external electronic device.

9. A method of controlling an electronic device, the method comprising:
displaying a first execution screen of a first application on a touchscreen display of the electronic device based on a chat session set between at least one external electronic device and the electronic device, wherein the first execution screen comprises a first visual element indicating a user of the at least one external electronic device and an exercise progress situation of a user of the electronic device, and a second visual element for outputting a guidance message for guiding exercise progress;
receiving a selection input for the second visual element; and
displaying a second execution screen of the first application on the touchscreen display based on the selection input, the second screen comprising at least one of the first visual element and the guidance message.

10. The method of claim 9, wherein the first execution screen further comprises a third visual element indicating a current exercise status of a user of the at least one external electronic device.

11. The method of claim 9, wherein the first execution screen further comprises a text message of the user of the at least one external electronic device received through the chat session and a fourth visual object indicating whether the user of the at least one external electronic device is receiving the guidance message.

12. The method of claim 9, wherein the first execution screen further comprises a fifth visual object indicating ranking information according to an exercise progress situation of the user of the at least one external electronic device.

13. The method of claim 9, further comprising:
identifying a difference between a predetermined target value and a current exercise status of the user of the electronic device, and providing information on the identified difference as the guidance message.

14. The method of claim 9, further comprising:
providing the guidance message based on ranking information according to exercise progress situations of the user of the at least one external electronic device and the user of the electronic device.

15. The method of claim 9, wherein the first execution screen comprises a chat room based on the chat session, the method further comprising:
wherein the method further comprises displaying a type of an exercise for generating the chat room and a third execution screen for setting at least one sensor for acquiring biometric information of the user according to the type of the exercise.
